# FASCICULE DE BREVET EUROPEEN

(11) **EP 3 831 445 B1**
(45) Date de publication et mention de la délivrance du brevet: **06.11.2024**
(21) Numéro de dépôt: 20211294.2
(22) Date de dépôt: 02.12.2020
(51) Int. Cl.: A61N 1/378, A61N 1/372, A61N 1/37, G08B 21/18, G01R 19/165, G01R 31/36, G16H 40/40, G16H 40/67

(54) **PROCÉDÉ ET DISPOSITIF ACTIF MÉDICAL IMPLANTABLE POUR DÉTERMINER LA CAPACITÉ UTILISABLE D'UNE BATTERIE D'UN TEL DISPOSITIF**
MEDIZINISCHES VERFAHREN UND GERÄT ZUR BESTIMMUNG DER NUTZBAREN KAPAZITÄT EINER BATTERIE EINES SOLCHEN GERÄTS
IMPLANTABLE MEDICAL PROCESS AND DEVICE FOR DETERMINING THE USABLE CAPACITY OF A BATTERY OF SUCH A DEVICE

(30) Priorité: 02.12.2019 FR 1913604
(43) Date de publication de la demande: 09.06.2021
(73) Titulaire: Sorin CRM SAS, 92140 Clamart (FR)
(72) Inventeur: BEGUIN, Emmanuel, 78180 Montigny-le-Bretonneux (FR)
(74) Mandataire: Ungerer, Olaf

(56) Documents cités:
- US-A- 4 715 381
- US-A1- 2010 010 559
- US-A1- 2012 109 248
- US-A1- 2014 277 286
- US-A1- 2015 105 842
- US-B2- 6 671 552

## Description

La présente invention se rapporte à un procédé ainsi qu'à un dispositif actif médical implantable, en particulier un stimulateur cardiaque ou un neurostimulateur, pour déterminer la capacité utilisable d'une batterie d'un dispositif actif médical implantable équipé d'une liaison radiofréquence (RF), notamment pour communiquer avec un autre dispositif, en particulier un autre dispositif externe non-implantable.

Les dispositifs actifs implantables peuvent comprendre une unité de communication radiofréquence (RF) pour permettre une communication avec un autre dispositif, en particulier un dispositif externe, par exemple un programmateur utilisé par un praticien, un home-monitor, un smartphone ou tout autre dispositif. Ici, on comprend par communication toute communication entre le dispositif implantable et un autre dispositif, indépendamment du fait qu'il y ait une réponse ou pas de l'autre dispositif.

Il est primordial pour les patients porteurs d'un dispositif actif médical implantable avec une fonctionnalité de communication par RF que la capacité utilisable de la batterie d'un tel dispositif puisse être déterminée. La batterie d'un dispositif médical implantable, hormis dans le cas d'un défibrillateur implantable, est soumise à des courants bien plus importants lors d'une communication RF que ne le demande la délivrance de la thérapie. Il est ainsi nécessaire de définir un indicateur qui déclenchera une alerte informant le personnel médical que la liaison RF ne pourra plus être utilisée. En effet, il est nécessaire que le praticien soit informé de l'épuisement de la fonctionnalité de communication RF de façon à ce qu'il puisse procéder au remplacement de l'appareil afin de pouvoir continuer à suivre le patient à distance.

Il s'avère que les données de la batterie fournies par son constructeur ne correspondent pas aux conditions réelles d'utilisation par la RF. De ce fait, elles ne sont pas utilisables telles quelles pour déterminer jusqu'où la capacité de la batterie est disponible pour la RF. En effet, traditionnellement, les informations fournies sont la tension au repos (ou sous faible charge) et la tension lorsque la batterie est soumise à un courant fort, par exemple de 5mA à 30mA, et stable et pendant une durée définie. Ces données peuvent être utilisées seules ou conjointement, mais elles ne sont pas applicables à un profil de courant dynamique lors d'une communication RF qui est caractérisé par des fortes fluctuations du courant liées au protocole de communication. S'en tenir à ces données conduit ainsi à prendre des marges considérables qui grèvent fortement la capacité utilisable, ou bien à surestimer la capacité utilisable au risque de compromettre à partir d'un certain niveau de déplétion le fonctionnement du dispositif actif médical implantable lors de l'utilisation de la RF.

Une autre méthode connue consiste à mesurer continuellement la tension ou l'impédance de la batterie lors d'une communication RF de façon à vérifier si elle atteint un seuil prédéterminé. La sollicitation de la batterie a lieu essentiellement lors des suivis à distance ou en présence d'un praticien. De ce fait, Il n'est pas adéquat d'attendre le prochain suivi du patient par communication RF pour savoir si l'état de la batterie permet encore d'utiliser la liaison RF. En effet, il peut se passer plusieurs mois, voire une année entre deux suivis. Cela conduirait à ce que les performances du dispositif actif médical implantable soient dégradées lorsque le suivi a lieu alors que la batterie n'est plus apte à supporter cette sollicitation, ou bien à interrompre la liaison RF dès que le seuil est atteint.

Lorsque la limite de l'utilisation de la liaison RF coïncide avec l'indicateur de remplacement (en anglais « Recommended Replacement Time », RRT), il est nécessaire de définir précisément la longévité résiduelle à chaque fois que le dispositif actif médical implantable est interrogé. Cet indicateur de longévité résiduelle repose sur une évaluation du niveau de déplétion de la batterie. Il y a donc nécessité d'avoir un indicateur fiable et disponible et mis à jour très régulièrement, par exemple quotidiennement ou hebdomadairement, tout en assurant au médecin et au patient une longévité optimisée pour l'utilisation de la RF. Les documents US 2012/109248 et US 2014/277286 décrivent des dispositifs et procédés associés connus de l'art.

L'objet de l'invention est de définir un procédé qui optimise la détermination de la capacité utilisable de la batterie d'un dispositif médical implantable actif permettant de communiquer par une liaison RF avec un programmateur, un home-moniteur ou tout autre dispositif, en particulier externe.

L'objet de la présente invention est atteint par un procédé selon la revendication 1 pour déterminer la capacité utilisable d'une batterie d'un dispositif actif médical implantable, en particulier un stimulateur cardiaque, comprenant une unité de communication radiofréquence (RF) pour transmettre des données pendant une durée de communication par RF, et dont la capacité utilisable de la batterie du dispositif actif médical implantable permet la transmission de données par RF par l'unité de communication RF.

L'invention prend avantage du fait que la baisse de tension instantanée au début d'une sollicitation donne suffisamment d'information pour déterminer la capacité de la batterie. Ce procédé permet ainsi d'augmenter la longévité de l'utilisation de la batterie d'un dispositif actif médical implantable, notamment en s'affranchissant des marges considérables déduites des données du constructeur qui grèvent fortement la capacité utilisable. La baisse instantanée observée est surtout liée à la partie résistive de la batterie.

La présente invention, relative à un procédé pour déterminer la capacité utilisable d'une batterie d'un dispositif actif médical implantable, peut davantage être améliorée grâce aux modes de réalisations suivants.

Selon un mode de réalisation, la sollicitation peut correspondre à un appel de courant d'une durée inférieure à 20ms, en particulier inférieure à 10 ms.

Ainsi, par l'application d'une impulsion de durée plus courte par rapport à la durée d'une communication nécessaire à la transmission de données, il est possible de déterminer la capacité utilisable de la batterie. De ce fait, il n'est pas nécessaire d'avoir recours à une transmission de données par RF complète pour déterminer la capacité utilisable, c'est-à-dire d'utiliser une communication RF lors des suivis à distance ou en présence d'un praticien, qui peuvent, de plus est, n'avoir lieu qu'une fois par an.

Selon un mode de réalisation, l'étape b) peut comprendre la mesure d'au moins une tension de crête haute et la tension de crête basse suivante de la tension de la batterie qui oscille entre des valeurs crête-crête lors d'une communication RF.

Ainsi, n'importe quel type de communication RF du dispositif implantable, par exemple l'« advertising » selon le protocole Bluetooth ou lors d'un échange régulier entre le dispositif et tout appareil externe, peut être utilisé pour déterminer l'état de la batterie. La détermination des valeurs haute et basse peut se faire à tout moment, car la baisse instantanée entre une valeur haute et la valeur basse suivante est liée de façon prépondérante à la partie résistive de la batterie qui pour un état donné de déplétion de batterie est essentiellement constante.

Selon un mode de réalisation, l'étape b) et peut comprendre en outre la mesure de la tension au repos de la batterie avant toute sollicitation.

Ainsi, même si les tensions de crêtes haute et basse sont mesurées en cours de la transmission, la mesure de tension au repos peut être utilisée pour normaliser la mesure pour pouvoir réaliser la comparaison avec le seuil de tension prédéterminé VBₛ.

Selon un mode de réalisation, l'appel de courant peut être d'un facteur d'au moins 100, en particulier d'au moins 1000, plus courte que la durée de transmission de données prédéterminée.

Ainsi, la batterie n'est que peu sollicitée pour déterminer sa capacité utilisable.

Selon un mode de réalisation, l'appel de courant peut être réalisée avec un courant crête, correspondant au courant maximum présent lors d'une transmission de données RF.

Ainsi, on se met dans une situation comparable à la transmission de données ce qui rend d'autant plus fiable le procédé pour déterminer si la batterie peut encore transmettre des données par RF.

Selon un mode de réalisation, l'étape de mesure de la tension VBᵢ de la batterie dudit dispositif actif médical implantable peut être réalisée de manière périodique, en particulier quotidiennement.

Ainsi, la tension VBᵢ de la batterie peut être mesurée plus régulièrement et la prédiction de longévité peut être évaluée et disponible à chaque interrogation, que celle-ci soit faite à distance ou en milieu hospitalier. De plus, comme il s'agit d'un appel de courant de courte durée, l'impact sur la longévité de la batterie est négligeable. En effet, il n'est bien entendu pas question de mesurer périodiquement la tension de la batterie soumise à un appel de courant de durée égale à une transmission de données par RF. Ainsi, la durée de vie du dispositif n'est pas impactée de manière substantielle.

Selon un mode de réalisation, le seuil de tension prédéterminé VBₛ peut représenter un seuil de tension permettant encore au moins une transmission de données par RF dudit dispositif actif médical implantable avec un autre dispositif, en particulier avec un autre dispositif externe non-implantable.

Ainsi, au moins un message d'alerte peut encore être transmis à un autre dispositif, en particulier un autre dispositif externe non-implantable. Il est ainsi possible de prévenir le personnel médical de ce stade de déplétion de la batterie.

Selon un mode de réalisation, le seuil de tension prédéterminé VBₛ peut être supérieur à une tension limite VB_{ref} représentative de la limite de fonctionnement du circuit de communication RF dudit dispositif actif médical implantable.

Ainsi, le procédé prévoit une marge de sécurité assurant au moins la transmission d'un message d'alerte avant que la liaison RF ne puisse plus être utilisée.

Selon un mode de réalisation, l'appel de courant peut être réalisé en utilisant l'unité RF dudit dispositif actif médical implantable.

Ainsi, l'invention peut être réalisée sans source supplémentaire dédiée au courant nécessaire pour l'appel de courant.

Selon un mode de réalisation, la génération de l'appel de courant peut comprendre la génération d'une onde porteuse accompagnée ou pas de l'activation d'au moins une partie des fonctions nécessaires pour la transmission de données RF.

De ce fait, le courant peut être fourni par l'unité RF en activant des éléments générant le courant crête requis lors d'une transmission de données RF. Ainsi, le procédé permet la génération d'un courant comparable à celui présent lors d'une transmission de données RF.

Selon un mode de réalisation, la génération de l'appel de courant comprend l'utilisation du canal de recherche de connexion pour initier une communication RF dudit dispositif actif médical implantable avec un autre dispositif, en particulier en utilisant le canal primaire d'advertising du protocole Bluetooth.

De ce fait, l'appel de courant peut être l'un de ceux déjà présents dans le protocole de recherche de connexion dans la mesure où le courant est représentatif du courant crête lors d'une communication RF. Cela assure également qu'aucune perturbation ne soit causée avec les communications présentes dans l'environnement du porteur du dispositif médical implantable.

Selon une variante, le procédé peut comprendre une étape de détermination de la capacité résiduelle et/ou de la longévité résiduelle de la batterie en fonction de la tension VBᵢ mesurée.

Ceci peut être réalisé en comparant la tension VBi avec des tables enregistrées dans la mémoire du dispositif actif médical. Pour déterminer la longévité résiduelle, l'usage de la batterie dans le passé peut être pris en compte.

Selon un mode de réalisation, le procédé peut comprendre la transmission d'un message en fonction du la capacité résiduelle et/ou de la longévité résiduelle de la batterie.

Ainsi, un message est transmis permettant à l'utilisateur ou le personnel traitant de savoir dans combien de temps la transmission de donnés par RF ne sera plus possible.

Selon un mode de réalisation, le procédé peut comprendre une étape de transmission de la valeur VBᵢ à un autre dispositif sur demande de l'autre dispositif et/ou de l'utilisateur Ainsi, la capacité résiduelle et/ou longévité résiduelle peut aussi être établie à l'extérieur du dispositif actif médical. Cela réduit le besoin en mémoire et en puissance de calcul du dispositif actif médical implanté permettant ainsi de réduire la consommation en énergie et le volume du dispositif.

Selon un mode de réalisation, le procédé peut comprendre une étape d'autorisation d'un suivi automatique ou pas en fonction de VBᵢ.

Selon l'état de la santé de l'utilisateur, le praticien peut programmer le dispositif médical implantable de manière à autoriser ou pas la transmission de données par RF. Pour un patient pour lequel la transmission n'est pas nécessaire, cette fonctionnalité n'est alors pas utilisée ce qui permet de prolonger la durée de vie de la batterie. Il se peut néanmoins que suite à une dégradation de l'état de santé de l'utilisateur, le praticien décide que la transmission automatique de données par RF soit utilisée. Si, à ce moment, la valeur de VBᵢ est déjà en dessous du seuil VBₛ, l'utilisation de la fonctionnalité RF sera bloquée pour éviter un malfonctionnement du dispositif.

Selon un mode de réalisation, la transmission de données RF du dispositif actif médical peut être suspendue suite au franchissement du seuil de tension prédéterminé VBₛ.

Ainsi le dispositif actif médical peut encore être utilisé sans cette fonctionnalité jusqu'à atteindre un autre paramètre, tel que l'indicateur de remplacement.

L'objet de la présente invention est également atteint par un dispositif actif médical implantable selon la revendicatiopn 18 et équipé d'une liaison RF comprenant une batterie, une unité RF et un contrôleur. Le contrôleur est configuré pour mettre en oeuvre le procédé tel que décrit ci-dessus.

Ainsi, par l'application d'une impulsion de durée plus courte par rapport à la durée d'une communication nécessaire à la transmission de données RF, il est possible de déterminer la capacité utilisable de la batterie. De ce fait, il n'est pas nécessaire d'avoir recours à une transmission de données par RF complète pour déterminer la capacité utilisable, c'est-à-dire d'utiliser une communication RF lors des suivis à distance ou en présence d'un praticien, qui peuvent, de plus, n'avoir lieu qu'une fois par an. Autrement dit, grâce au dispositif actif médical implantable inventif, il n'y a pas besoin d'établir une communication par RF pour déterminer si une transmission de données est encore possible. De plus, comme il s'agit d'un appel de courant de durée plus courte que celle d'une transmission de données par RF, l'impact sur la longévité de la batterie est négligeable. Il est ainsi possible d'augmenter la longévité de l'utilisation de la batterie du dispositif actif médical implantable, notamment en s'affranchissent des marges considérables déduites des données du constructeur qui grèvent fortement la capacité utilisable.

L'invention et ses avantages seront expliqués plus en détail dans la suite au moyen de modes de réalisation préférés et en s'appuyant notamment sur les figures d'accompagnement suivantes, dans lesquelles :
La figure 1 représente un organigramme d'un procédé selon la présente invention pour déterminer la capacité utilisable d'une batterie d'un dispositif actif médical implantable ;
La figure 2 représente un dispositif actif médical implantable selon la présente invention ;
La figure 3 illustre un graphe représentant la tension dans la batterie soumis aux appels de courant lors d'une transmission de données RF ;
La figure 4 illustre un graphe représentant la tension d'une batterie en fonction du courant moyen lorsqu'elle est soumise à des appels de courant pour une durée représentative d'une transmission de données RF;
La figure 5 illustre un graphe représentant la tension d'une batterie en fonction du courant moyen lorsqu'elle est soumise à des appels de courant pour une durée représentative d'une transmission de données RF et la détermination de dVBᵢ quand la mesure n'est pas faite au début d'une transmission de données RF.

Pour assurer que la batterie d'un dispositif actif médical implantable avec une fonctionnalité de communication radiofréquence RF soit toujours en capacité de fournir la puissance nécessaire à la transmission de données RF, l'invention propose un procédé qui permet de vérifier régulièrement l'état de la batterie, par exemple quotidiennement, sans trop solliciter la batterie elle-même. Les conditions de transmission RF sont typiquement prédéterminées, ainsi le débit de la transmission, la taille de la zone mémoire à transmettre et, la durée de la communication sont connus. Il existe différents types de communication RF comme par exemple un réveil périodique avec une communication brève d'une durée de quelques millisecondes à plusieurs dizaines de millisecondes pour vérifier s'il y a besoin ou pas de réaliser une communication (par exemple, en utilisant le mode « advertising » du standard Bluetooth). Une communication RF peut également servir pour envoyer un message afin d'indiquer si oui ou non il faut communiquer avec un autre dispositif. Il peut s'agir d'une communication avec transmission de données d'une durée inférieure à une seconde. Finalement, il peut s'agir d'une communication avec transmission de données qui peut durer plusieurs dizaines de secondes.

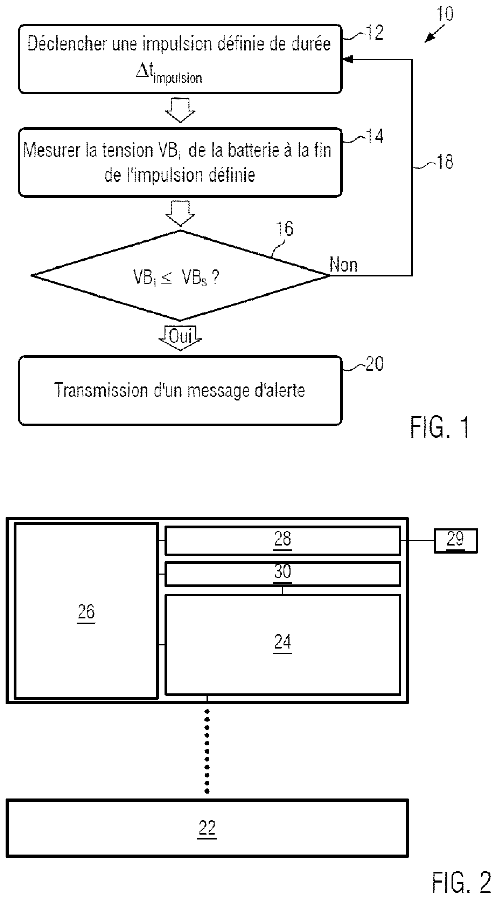

Le procédé selon un mode de réalisation de la présente invention pour déterminer la capacité utilisable d'une batterie d'un dispositif actif médical implantable sera décrit au moyen de l'organigramme de la Figure 1.

Lors d'une première étape 12 du procédé 10 selon la présente invention, un appel de courant défini de durée Δtᵢₘₚᵤₗₛᵢₒₙ et d'intensité représentative d'un courant crête requis par la transmission de données par RF d'un dispositif actif médical implantable est déclenché. L'appel de courant a une durée Δtᵢₘₚᵤₗₛᵢₒₙ inférieure à 20ms, en particulier inférieure à 10ms pour être suffisamment courte pour essentiellement correspondre à la baisse instantanée de la tension de batterie.

Ainsi l'appel de courant a une durée plus courte que la durée de la transmission de données RF. Pour une transmission de données RF typique d'un stimulateur cette durée est plus courte d'au moins un facteur de 100 fois, plus en particulier d'un facteur d'au moins 1000 fois.

Lors d'une deuxième étape 14 du procédé 10 selon l'invention, la tension VBᵢ de la batterie est mesurée à la fin de l'appel de courant déclenché à l'étape 12. Cette tension est représentative de la baisse instantanée de la tension de la batterie suite à la sollicitation de la batterie.

Lors d'une troisième étape 16 du procédé 10 selon la présente invention, la tension VBᵢ mesurée est comparée à un seuil de tension prédéterminé de la batterie VBₛ.

Si la tension VBᵢ mesurée à la fin de l'appel de courant est supérieure au seuil de tension prédéterminé de la batterie VBₛ, cela signifie que la batterie du dispositif actif médical implantable a encore de la capacité utilisable pour assurer des transmissions de données par RF. Dès le franchissement du seuil, une transmission de données par RF complète ne peut plus être garantie. Telle qu'indiquée par la flèche 18, le procédé 10 revient alors à l'étape 12. L'étape 12 peut être répétée à des intervalles rapprochés, par exemple une fois par jour.

Notons que la valeur du seuil de tension prédéterminé de la batterie VBₛest fixée tout au long de la durée de vie de la batterie. Il n'est ainsi pas nécessaire de déterminer à nouveau la valeur du seuil de tension prédéterminé de la batterie VBₛ.

Dans le cas où la tension VBᵢ mesurée à la fin de l'appel de courant est inférieure ou égale au seuil de tension prédéterminé de la batterie VBₛ, un message d'alerte est transmis lors d'une l'étape 20 afin de prévenir le personnel médical ou l'utilisateur que la batterie a atteint le seuil de tension prédéterminé de la batterie VBₛ, nécessitant ou pas un échange du dispositif médical implantable selon que le seuil VBₛ coïncide ou pas avec l'indicateur de remplacement.

Selon une variante, la tension VBᵢ peut servir de référence pour déterminer la capacité résiduelle de la batterie pour l'utilisation de la RF, et donc servir de base à la détermination de la longévité résiduelle pour l'utilisation de la RF, avant d'atteindre la tension VBₛ.

Etant donné que la tension VBᵢ est mesurée régulièrement, par exemple quotidiennement, cette longévité résiduelle est disponible et actualisée à chaque fois que le dispositif actif médical implantable est interrogé, que cela soit à distance ou bien en présence d'un praticien. Ainsi, la valeur VBi peut être transmise à un autre dispositif, qui peut en fonction de tables ou d'un algorithme déterminer la capacité résiduelle et/ou la longévité résiduelle. Selon une variante, la capacité résiduelle ou la longévité résiduelle peuvent aussi être déterminées dans le dispositif actif médical.

A noter que si l'arrêt de l'utilisation de la RF coïncide avec le moment recommandé de remplacement (en anglais « Recommended Replacement Time »), la tension VBi peut servir de référence pour l'indicateur de longévité résiduelle du dispositif actif médical implantable.

Si l'arrêt de l'utilisation de la RF ne coïncide pas avec le moment recommandé de remplacement, le franchissement du seuil va entraîner l'arrêt de la fonctionnalité RF. Toutefois, le dispositif actif médical peut encore être utilisé jusqu'au moment recommandé de remplacement. Si l'état de santé de l'utilisateur le permet le dispositif peut alors encore être utilisé ce qui repousse donc le moment d'un changement du dispositif.

Selon un autre mode d'utilisation de la tension VBᵢ, l'atteinte de la tension VBₛ peut également servir à ne pas autoriser la programmation du suivi à distance pour le futur pour un dispositif qui ne l'était pas précédemment.

Comme il n'est pas nécessaire de mesurer périodiquement la tension de la batterie soumise à un appel de courant de durée égale à une transmission de données RF, la détermination de la capacité utilisable impacte moins la durée de vie de la batterie. En effet, comme il s'agit d'un appel de courant de courte durée par rapport à la durée d'une transmission de données RF, l'impact d'un tel appel de courant sur la longévité de la batterie est négligeable. De plus, le procédé 10 permet d'effectuer une mesure périodique, par exemple quotidienne, et permet de remplacer le dispositif actif médical implantable en cas de besoin sans urgence.

La présente invention se rapporte également à un dispositif actif médical implantable équipé d'une liaison RF comprenant une batterie, une unité RF et un contrôleur. Le contrôleur est configuré pour déterminer la capacité utilisable de la batterie selon le procédé 10 tel que décrit ci-dessus en référence à la Figure 1.

La Figure 2 illustre un tel dispositif actif médical implantable 20 configuré selon l'invention. Le dispositif actif médical implantable 20 est configuré pour pouvoir réaliser une liaison de communication sans fil, de type radiofréquence RF, avec un autre dispositif, en particulier avec un autre dispositif externe 22. Le dispositif médical implantable 20 peut être un stimulateur cardiaque ou un neurostimulateur. L'autre dispositif, en particulier l'autre dispositif externe 22, peut être un programmateur utilisé par un praticien clinique, un home-moniteur ou tout autre dispositif.

Le dispositif actif médical implantable 20 comprend une unité radiofréquence (RF) 24 permettant la communication avec l'autre dispositif, en particulier avec l'autre dispositif externe 22. Il comprend une batterie 26 pour permettre la communication RF. La batterie 26 fournit également l'énergie nécessaire pour délivrer une thérapie réalisée par un contrôleur 28 qui pilote une ou plusieurs électrodes 29. De plus, le dispositif actif médical implantable 20 comprend un contrôleur 30 configuré pour déterminer l'état de la batterie 26 selon le procédé 10 décrit ci-dessous. Ainsi, l'état de la batterie 26 est déterminé au moyen d'un appel de courant court de durée Δtᵢₘₚᵤₗₛᵢₒₙ d'une longueur inférieure à 20 ms, en particulier inférieure à 10ms, pour être inférieure à la durée d'une transmission de données RF habituelle du dispositif 20 et utilisée pour transmettre des données à l'autre dispositif, en particulier à l'autre dispositif externe 22.

Selon une variante avantageuse, l'appel de courant peut être réalisé en utilisant l'unité RF 24 dudit dispositif actif médical implantable 20. Dans ce cas, le courant est fourni par l'unité RF 24 en activant des éléments générant le courant crête requis lors d'une transmission de données RF. L'intérêt est de générer un courant identique, et de ne pas avoir à faire appel à une source de courant supplémentaire. Le courant crête est obtenu en générant l'onde porteuse et en activant les autres fonctions nécessitant un courant significatif pendant une transmission de données RF.

Selon une autre variante, le contrôleur 30 est configuré pour utiliser le protocole de communication en particulier le protocole de recherche de connexion, pour générer l'appel de courant. De ce fait, le procédé 10 peut être réalisé avec les fonctionnalités déjà disponibles dans le dispositif actif médical implantable 20.

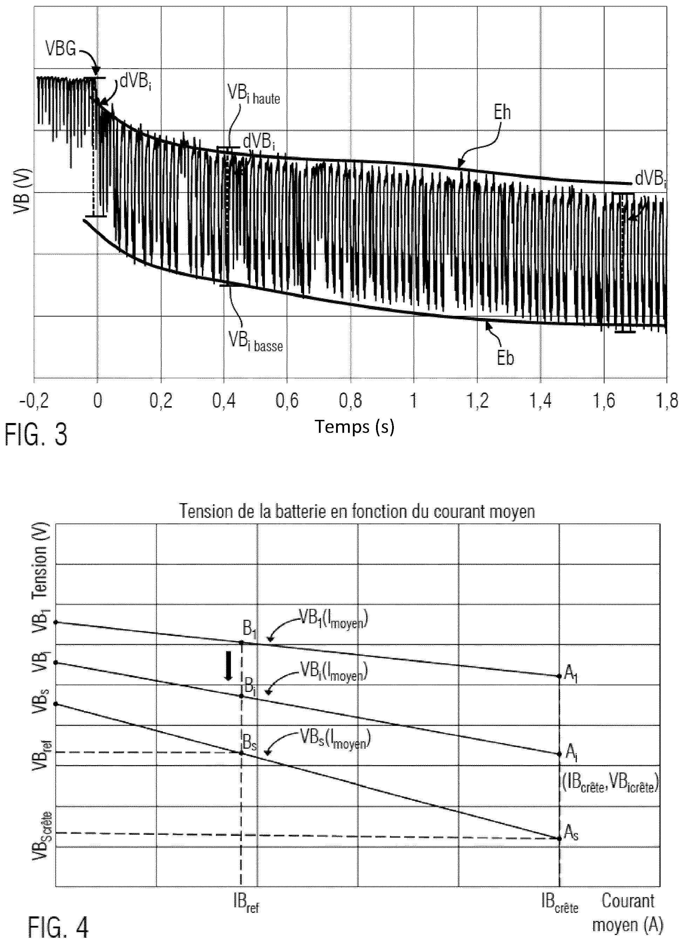

La Figure 3 illustre un graphe représentant la tension d'une batterie soumise à des appels de courant en fonction du temps et représentatif d'un début d'une transmission de données RF.

Le comportement d'une batterie quand elle est soumise à un régime d'appels de courant dynamiques d'une transmission de données RF, par exemple en utilisant le protocole Bluetooth ou tout autre protocole de communication RF adapté se traduit par une baisse de tension instantanée sur chaque appel de courant, plus au moins prononcée en fonction de la capacité de filtrage généralement connectée à la batterie. Cette baisse instantanée est liée de façon prépondérante à la partie résistive de la batterie qui, pour un état donné de déplétion de la batterie, peut être considérée constante.

Tel que le montre la Figure 3, la tension de la batterie VB oscille entre une tension crête haute VBᵢₕₐᵤₜₑ et une tension crête basse suivante VB_{ibasse} lors d'une transmission de données RF.

On constate également une baisse progressive au fur et à mesure de la communication RF ou la transmission des données RF. Le profil de la tension de la batterie est donc lié à un courant plus fort, appelé également courant crête qui crée ces baisses de tension instantanées, et à un courant moyen fourni pour réaliser la transmission de données RF, qui est à la base de la baisse de la tension progressive.

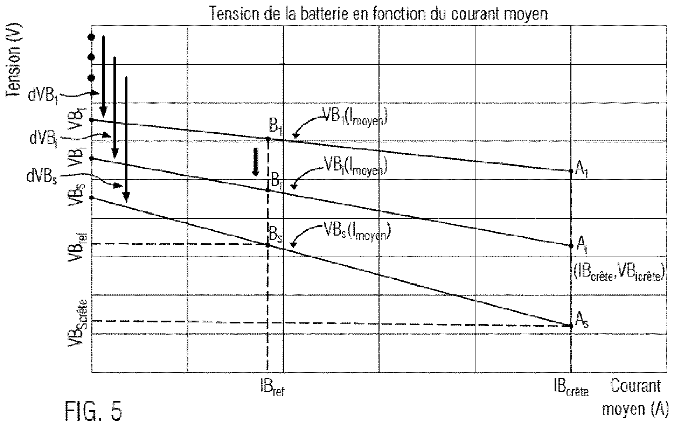

La baisse de tension relative instantanée notée dVBᵢ sur la Figure 3 se retrouve à chaque appel de courant tout au long de la transmission de données RF. Ainsi, au moment de la sollicitation de la batterie à l'instant t=0, la baisse instantanée dVBᵢ est la même qu'un peu plus tard par exemple vers 0,4s ou vers 1,65s. La tension de la batterie atteinte à la fin d'une transmission de données RF dépend donc du courant instantané responsable des baisses instantanées et du courant moyen responsable de la baisse progressive. Deux paramètres peuvent ainsi être définis, le premier paramètre, illustré sur la Figure 3, étant la tension de la batterie à la fin d'un seul appel de courant court représentatif de la baisse de tension instantanée dVBᵢ lors d'un courant crête, le deuxième paramètre étant la tension à la fin d'un seul appel de courant de durée prédéfinie qui est égale à la transmission de données RF et de courant égal au courant crête, qui va être expliqué plus en détail en relation avec les figures 4 et 5.

Ici, pour le premier paramètre, on comprend par appel de courant de courte durée une durée au moins égale à la durée des appels de courant qui sont responsables de la baisse instantanée de la tension de batterie, soit de l'ordre de 1 ms et au plus à une durée qui n'excède pas 20ms. Au-delà la baisse de tension devient plus conséquente et de moins en moins représentative.

Des valeurs typiques d'un courant crête maximal lors d'une transmission de données RF d'un dispositif actif médical implantable, tel qu'un stimulateur cardiaque, sont de l'ordre de 5mA à 30mA, un courant moyen est de l'ordre de 2mA à 10mA et la durée d'une communication RF pour un transfert complet de données stockées dans la mémoire d'un implant est de l'ordre de 10s à 60s.

La Figure 4 illustre un graphe représentant la tension de la batterie à la fin d'une transmission de données RF pour une durée définie en ordonnée en fonction de l'intensité du courant moyen tiré sur la batterie lors de la transmission de données RF en abscisse et en fonction de la déplétion de la batterie. La relation entre la tension à la fin de la transmission de données RF, et le courant moyen est en première approximation linéaire.

Sur la Figure 4 la tension VBᵢ avec i= 1 à n, correspond à la tension de la batterie après la baisse instantanée de la tension suite à la sollicitation de la batterie.

Pour l'appel de courant court, le courant ramené à la durée d'une transmission de données RF est négligeable, la baisse instantanée peut donc être placée à l'origine de l'abscisse. VBᵢ correspond ainsi à la tension observée à la fin de l'appel de courant court défini de durée Δtᵢₘₚᵤₗₛᵢₒₙ.

A l'inverse plus le courant moyen est fort, et plus il se rapproche du courant crête. Par approximation linéaire, la droite est prolongée jusqu'au point correspondant au courant crête (IB_{crête}). Ce point correspond donc à un appel de courant de la durée de la transmission de données RF dont le courant est constant et égale au courant crête.

Le point Aᵢ i= 1 à n de coordonnées (IB_{crête}; VB_{icrête}) sur le graphe de la Figure 4 correspond à l'appel de courant de durée égale à celle d'une transmission de données RF, avec le courant moyen de la transmission égal au courant crête iB_{crête}, c'est-à-dire au courant instantané maximum que la batterie fournit pendant toute la durée prédéterminée d'une transmission de données RF.

Selon le paramétrage de la communication RF du dispositif, le courant moyen se trouve donc entre les deux valeurs limites l=0 et l= iB_{cr}êtₑ. La tension de la batterie va se trouver, à première approximation, sur la droite reliant les deux points extrêmes Aᵢ et (0, VBᵢ).

En fonction de la déplétion de la batterie, la courbe VBᵢ(l_{moyen}) se déplace au fur et à mesure vers le bas, telle qu'indiquée par la flèche et une deuxième droite définie par les points Aᵢ et (0, VBi). On observe que la baisse de la tension VB_{icrête} en fonction de la déplétion est plus forte que la baisse de la tension VBᵢ. Ceci implique nécessairement qu'à partir d'un certain niveau de déplétion une tension limite de la batterie sera atteinte pour laquelle le fonctionnement de la transmission de données RF ne sera plus garantie. Une prédiction du bon et fiable fonctionnement du dispositif actif médical implantable et avec une optimisation de la capacité utilisable de la batterie est ainsi l'objet de l'invention.

L'invention se base sur le fait que le constructeur de la batterie est à même de fournir l'évolution de la tension de batterie pour un courant crête donné et les durées correspondant à l'appel de courant court et à la durée déterminée correspondant à celle de la transmission de données, donc les valeurs VBᵢ et Aᵢ. Grâce à ces données du constructeur, il est possible d'obtenir les droites VBᵢ(l_{moyen}) illustrées sur la figure 4 en fonction de la déplétion de la batterie. De plus, il devient possible de définir le niveau de déplétion de la batterie pour lequel la droite définie par As et VBₛ passe par le point Bs correspondant à la tension limite pour laquelle le fonctionnement de la transmission de données RF est garanti (VB_{ref}) et le courant moyen (IB_{ref}).

Le courant moyen IB_{ref} pour une transmission de données RF type du stimulateur peut être déterminé de manière expérimentale. De plus, le design du dispositif médical implantable donné permet également de définir une tension limite VB_{ref} nécessaire au bon fonctionnement et notamment pour assurer la liaison RF. Cette tension peut également être déterminée expérimentalement. Ces valeurs déterminent donc le point Bₛ, illustré dans la Figure 4.

En considérant la dépendance linéaire entre tension et courant moyen, l'extrapolation de la droite VBₛ(l_{moyen}) passant par les points Aₛ et Bₛ vers l'origine de l'abscisse va donner la tension limite VBₛ à ne pas franchir après l'application d'un appel de courant court.

Il suffit donc de comparer la tension VBᵢ représentant la tension de la batterie mesurée à la fin d'un appel de courant court de durée courte Δtᵢₘₚᵤₗₛᵢₒₙ au seuil de la tension limite VBₛ de la batterie afin de déterminer si la capacité utilisable de la batterie est encore suffisante. Comme la sollicitation de la batterie est très courte pour pouvoir déterminer VBᵢ, le test peut être réalisé de manière régulière et rapprochée, par exemple quotidiennement.

Etant donné l'énergie requise, Il n'est pas envisageable de mesurer périodiquement VB_{icrête}, afin de prendre en compte le point Aₛ pour chaque batterie utilisée dans un dispositif actif médical implantable pour pouvoir déterminer la tension seuil VBₛ, il est plus avantageux d'établir un seuil commun pour une pluralité de batteries pour des dispositifs actifs médicaux implantables de même type. Ainsi, il n'est pas nécessaire de déterminer VBscrête à nouveau pour chaque dispositif.

Par soucis de sécurité, la tension VB_{Scrête} choisie pour établir la droite VBₛ(l_{moyen}), correspond à la tension VB_{crête} de plus faible tension pour un même courant crête IB_{crête} observée pour une série de batterie de même type. Autrement dit, la tension VBscrête prise en compte en tant que référence peut correspondre à la batterie du « pire cas » afin d'introduire une marge de sécurité. Cette batterie aura une droite VBₛ(l_{moyen}), qui passera par le point Bs et qui aura la plus forte décroissance.

Le seuil de tension de la batterie VBₛ défini par la droite de référence VBₛ(l_{moyen}) sert de point de référence à l'ensemble des batteries. Du fait de la marge de sécurité introduite par la droite de référence VBₛ(l_{moyen}), le seuil de tension de la batterie VBₛ permet de ne pas atteindre complètement la tension limite de fonctionnement VB_{ref}de la batterie au moment où le seuil de tension VBₛ est atteint lors de la mesure sur l'appel de courant court. En effet, en fonction du niveau de déplétion de la batterie, la tension de la batterie mesurée VBᵢ à la fin d'un appel de courant court va diminuer depuis la valeur VB₁ vers la valeur VBₛ qui assure que la tension de batterie atteinte à la fin d'une transmission de données par RF reste au-dessus de VB_{ref}. Des droites successives reliant les points Aᵢ et point (0 ; VBᵢ) se déplacent de manière à descendre vers l'axe des abscisses et à se rapprocher de la droite de référence VBₛ(l_{moyen}) en fonction de la déplétion de la batterie. Au moment d'atteindre le seuil de tension VBₛ encore au moins une transmission de données par RF du dispositif actif médical implantable avec un autre dispositif, en particulier avec un autre dispositif externe non-implantable, sera possible. Il peut s'agir de préférence d'une transmission de données RF permettant de transmettre toute information utile et notamment la transmission des données présentes dans la mémoire. Selon une alternative il peut également s'agir d'un message d'alerte afin de prévenir le personnel médical de ce stade de déplétion de la batterie.

De ce fait, la marge de sécurité introduite par la droite de référence VBₛ(l_{moyen}) assure au moins la transmission de données RF ou d'un message d'alerte avant que la liaison RF ne puisse plus être utilisée. On notera que la tension VBₛ sert de référence pour le seuil de tension prédéterminé pour une pluralité de dispositifs actifs médicaux implantables. Il n'est donc pas nécessaire de redéfinir VBₛ pour chaque dispositif.

La figure 5 illustre un graphe représentant la tension d'une batterie en fonction du courant moyen lorsqu'elle est soumise à des appels de courant pour une durée représentative d'une transmission de données RF et la détermination de dVBᵢ quand la mesure n'est ni faite au début d'une transmission de données RF ni au moyen d'une sollicitation de courant spécifique à la mesure VBᵢ.

Comme déjà décrit ci-dessus, la baisse de tension instantanée notée dVBᵢ sur la Figure 3 se retrouve à chaque appel de courant tout au long de la transmission de données RF. Ainsi, au moment de la sollicitation de la batterie à l'instant t=0, la baisse instantanée dVBᵢ est la même qu'un peu plus tard vers 0,4s ou vers 1,65s.

Ainsi, la méthode telle que décrite par rapport aux figures 1 et 2 peut, selon une alternative, également être réalisée avec n'importe quelle baisse instantanée lors d'une transmission de données RF. En mesurant une tension crête haute VBᵢₕₐᵤₜₑ et la tension crête basse suivante VB_{ibasse} de la tension de la batterie VB qui oscille entre des valeurs hautes et basses lors d'une transmission de données RF, par exemple vers 0,4s sur la Figure 3, la valeur dVBᵢ peut aussi être déterminée. Par contre, il faut également prendre en compte la valeur VBGᵢ, la tension au repos de la batterie au moment de la mesure, pour pouvoir obtenir la valeur VBᵢ. En effet, VBᵢ= VBGᵢ - (VBᵢₕₐᵤₜₑ - VB_{ibasse}).

Telle que montrée sur la figure 3, la tension au repos VBGᵢ baisse également en fonction de l'état de déplétion de la batterie.

Pour réduire la marge d'erreur il est également possible de déterminer dVBᵢ à plusieurs moments de la transmission de données RF ou de déterminer une enveloppe Eₕ qui passe par les valeurs hautes et une autre enveloppe E_{b} qui passe par les valeurs basses et de déterminer la distance entre les deux.

## Revendications

1. Un procédé pour déterminer la capacité utilisable d'une batterie d'un dispositif actif médical implantable, en particulier un stimulateur cardiaque, comprenant une unité de communication radiofréquence (RF) pour transmettre des données pendant une durée de transmission par RF, et
dont la capacité utilisable de la batterie du dispositif actif médical implantable permet la transmission de données par RF par l'unité de communication RF, le procédé comprenant :
a) une étape de déclenchement d'un appel de courant défini d'une durée et d'une intensité représentative d'un courant crête requis par la transmission de données par RF du dispositif actif médical implantable ;
b) une étape de mesure d'une valeur de tension VBᵢ de la batterie représentative d'une baisse instantanée de tension dVBᵢ de la batterie dudit dispositif consécutive à une sollicitation en courant de la batterie correspondant à l'appel de courant ;
c) une étape de comparaison de ladite tension VBᵢ de la batterie à un seuil de tension prédéterminé VBₛ; et
d) une étape de transmission d'un message d'alerte à un autre dispositif lorsque ladite tension mesurée VBᵢ de la batterie franchit le seuil de tension prédéterminé VBₛ.

2. Le procédé selon la revendication 1, dans lequel la sollicitation correspond à l'appel de courant d'une durée inférieure à 20 ms, en particulier inférieure à 10 ms.

3. Le procédé selon la revendication 1 ou 2, dont l'étape b) comprend la mesure d'au moins une tension de crête haute et la tension de crête basse suivante de la tension de la batterie qui oscille entre des valeurs crête-crête lors d'une transmission RF.

4. Le procédé selon la revendication 3, dont l'étape b) comprend en outre la mesure de la tension au repos de la batterie avant toute sollicitation.

5. Le procédé selon l'une des revendications 2 à 4, dans lequel l'appel de courant est plus court d'un facteur d'au moins 100, plus en particulier d'au moins 1000, à la durée de transmission de donnée prédéterminée.

6. Le procédé selon l'une des revendications 2 à 5, dans lequel l'appel de courant est réalisé avec un courant crête, correspondant au courant maximum présent lors d'une transmission de données RF.

7. Le procédé selon l'une des revendications 1 à 6, dont les étapes a) à d) sont réalisées de manière périodique, en particulier quotidiennement.

8. Le procédé selon l'une des revendications 1 à 7, dont le seuil de tension prédéterminé VBₛ représente un seuil de tension permettant encore au moins une transmission par RF dudit dispositif actif médical implantable avec un autre dispositif.

9. Le procédé selon l'une des revendications précédentes, dont le seuil de tension VBₛ prédéterminé est supérieur à une tension limite VB_{ref} représentative de la limite de fonctionnement du circuit de communication RF dudit dispositif actif médical implantable.

10. Le procédé selon l'une des revendications 2 à 9, dont l'appel de courant est réalisé en utilisant l'unité RF dudit dispositif actif médical implantable.

11. Le procédé selon la revendication 10, dont la génération de l'appel de courant comprend la génération d'une onde porteuse accompagnée ou pas de l'activation d'au moins une partie des fonctions nécessaires pour la transmission de données RF.

12. Le procédé selon la revendication 10 ou 11, dans lequel la génération de l'appel de courant comprend l'utilisation d'un canal de recherche de connexion pour initier une communication RF dudit dispositif actif médical implantable avec un autre dispositif, en particulier en utilisant un canal primaire du protocole Bluetooth.

13. Le procédé selon l'une des revendications 1 à 12, comprenant une étape de détermination de la capacité résiduelle et/ou d'une estimation de longévité résiduelle de la batterie en fonction de la tension VBᵢ mesurée.

14. Le procédé selon la revendication 13, comprenant en plus la transmission d'un message en fonction de la capacité résiduelle et/ou de l'estimation de longévité résiduelle.

15. Le procédé selon une des revendications 1 à 14, comprenant une étape de transmission de la valeur VBᵢ à un autre dispositif sur demande de l'autre dispositif et/ou de l'utilisateur.

16. Le procédé selon l'une des revendications 1 à 15, comprenant une étape d'autorisation d'un suivi automatique ou pas en fonction de VBᵢ.

17. Le procédé selon l'une des revendications 1 à 16, dans lequel la transmission de données RF du dispositif actif médical est suspendue à la suite du franchissement du seuil de tension prédéterminé VBₛ.

18. Un dispositif actif médical implantable, en particulier un stimulateur cardiaque, comprenant une unité radiofréquence RF (24), une batterie (26) et un contrôleur (30) ; le contrôleur (30) étant configuré pour mettre en oeuvre le procédé (10) selon au moins l'une des revendications 1 à 17.

## Patentansprüche

1. Verfahren zur Bestimmung der nutzbaren Kapazität einer Batterie einer implantierbaren aktiven medizinischen Vorrichtung, insbesondere eines Herzschrittmachers, mit einer Hochfrequenz-(HF)-Kommunikationseinheit zur Übertragung von Daten während einer HF-Übertragungszeit, und
wobei die nutzbare Kapazität der Batterie der implantierbaren aktiven medizinischen Vorrichtung die Übertragung von Daten mittels HF durch die HF-Kommunikationseinheit ermöglicht, wobei das Verfahren umfasst:
a) einen Schritt des Auslösens eines durch eine Dauer und Intensität definierten Strombedarfs, der für einen für die Datenübertragung der implantierbaren aktiven medizinischen Vorrichtung mittels HF erforderlichen Spitzenstrom repräsentativ sind;
b) einen Schritt des Messens eines Batteriespannungswerts VBᵢ, der für einen momentanen Abfall der Spannung dVBᵢ der Batterie der Vorrichtung nach einer Strombelastung der Batterie entsprechend dem Strombedarf repräsentativ ist;
c) einen Schritt des Vergleichens der Spannung VBᵢ der Batterie mit einer vorbestimmten Spannungsschwelle VBₛ; und
d) einen Schritt des Sendens einer Warnmeldung an eine andere Vorrichtung, wenn die gemessene Spannung VBᵢ der Batterie die vorgegebene Spannungsschwelle VBₛ überschreitet.

2. Verfahren nach Anspruch 1, wobei die Belastung einem Strombedarf für eine Dauer von weniger als 20 ms, insbesondere weniger als 10 ms, entspricht.

3. Verfahren nach Anspruch 1 oder 2, wobei Schritt b) das Messen mindestens einer hohen Spitzenspannung und der nächsten niedrigen Spitzenspannung der Batteriespannung umfasst, die während einer HF-Übertragung zwischen Spitze-Spitze-Werten oszilliert.

4. Verfahren nach Anspruch 3, wobei der Schritt b) ferner das Messen der Ruhespannung der Batterie vor einer Belastung umfasst.

5. Verfahren nach einem der Ansprüche 2 bis 4, wobei der Strombedarf um einen Faktor von mindestens 100, insbesondere mindestens 1000, kürzer ist als die vorbestimmte Datenübertragungszeit.

6. Verfahren nach einem der Ansprüche 2 bis 5, wobei der Strombedarf mit einem Spitzenstrom realisiert wird, der dem maximal vorhandenen Strom während einer HF-Datenübertragung entspricht.

7. Verfahren nach einem der Ansprüche 1 bis 6, wobei die Schritte a) bis d) periodisch, insbesondere täglich, durchgeführt werden.

8. Verfahren nach einem der Ansprüche 1 bis 7, wobei die vorbestimmte Spannungsschwelle VBₛ eine Spannungsschwelle darstellt, die mindestens noch eine weitere HF-Übertragung der implantierbaren aktiven medizinischen Vorrichtung mit einer anderen Vorrichtung ermöglicht.

9. Verfahren nach einem der vorhergehenden Ansprüche, wobei die vorbestimmte Spannungsschwelle VBₛ höher ist als eine Grenzspannung VB_{ref}, die für die Betriebsgrenze der HF-Kommunikationsschaltung der implantierbaren aktiven medizinischen Vorrichtung repräsentativ ist.

10. Verfahren nach einem der Ansprüche 2 bis 9, wobei der Strombedarf durch Verwenden der HF-Einheit der implantierbaren aktiven medizinischen Vorrichtung realisiert wird.

11. Verfahren nach Anspruch 10, wobei die Erzeugung des Strombedarfs die Erzeugung einer Trägerwelle umfasst, die von der Aktivierung zumindest eines Teils der für die HF-Datenübertragung erforderlichen Funktionen begleitet ist oder nicht.

12. Verfahren nach Anspruch 10 oder 11, wobei die Erzeugung des Strombedarfs die Verwendung eines verbindungssuchenden Kanals umfasst, um die HF-Kommunikation der implantierbaren aktiven medizinischen Vorrichtung mit einer anderen Vorrichtung zu initiieren, insbesondere unter Verwendung eines primären Kanals des Bluetooth-Protokolls.

13. Verfahren nach einem der Ansprüche 1 bis 12, umfassend einen Schritt des Bestimmens der Restkapazität und/oder einer Schätzung der Restlebensdauer der Batterie in Abhängigkeit der gemessenen Spannung VBᵢ.

14. Verfahren nach Anspruch 13, ferner umfassend das Übertragen einer Nachricht auf Grundlage der Restkapazität und/oder der Schätzung der Restlebensdauer.

15. Verfahren nach einem der Ansprüche 1 bis 14, umfassend einen Schritt des Übertragens des Werts VBᵢ an eine andere Vorrichtung auf Anfrage der anderen Vorrichtung und/oder des Benutzers.

16. Verfahren nach einem der Ansprüche 1 bis 15, umfassend einen Schritt des Autorisierens oder nicht einer automatischen Überwachung in Abhängigkeit von VBᵢ.

17. Verfahren nach einem der Ansprüche 1 bis 16, wobei die HF-Datenübertragung von der aktiven medizinischen Vorrichtung nach Überschreiten der vorbestimmten Spannungsschwelle VBₛ unterbrochen wird.

18. Implantierbare aktive medizinische Vorrichtung, insbesondere Herzschrittmacher, umfassend eine HF-Funkeinheit (24), eine Batterie (26) und eine Steuerung (30); wobei die Steuerung (30) so konfiguriert ist, dass sie das Verfahren (10) gemäß mindestens einem der Ansprüche 1 bis 17 ausführt.

## Claims

1. A method for determining usable capacity of a battery of an active implantable medical device, in particular a cardiac stimulator, comprising a radiofrequency (RF) communication unit for transmitting data by RF over a transmitting period, and wherein the usable capacity of the battery of the active implantable medical device allows for data transmission by RF via the RF communication unit, the method comprising:
a) a step for triggering a current draw being defined with a duration and an intensity which is representative of a peak current required by the data transmission by RF from the active implantable medical device;
b) a step for measuring a value of the voltage VBᵢ of the battery which is representative of an instantaneous voltage drop dVBᵢ of the battery of said device as a result of a current load on the battery corresponding to the current draw thereon;
c) a step for comparing said voltage VBᵢ of the battery with a predetermined threshold voltage VBₛ; and
d) a step for transmitting an alert message to another device when said measured voltage VBᵢ of the battery crosses the predetermined threshold voltage VBₛ.

2. The method of claim 1, wherein the load corresponds to the current draw with a period of less than 20 ms, in particular of less than 10 ms.

3. The method of claim 1 or claim 2, wherein step b) comprises measuring at least one high peak voltage and the next low peak voltage of the battery voltage which oscillates between peak-to-peak values during the RF communication.

4. The method of claim 3, wherein step b) further comprises measuring the open circuit voltage of the battery before any loading.

5. The method of any one of claims 2 to 4, wherein the current draw is shorter than a predetermined duration of data transmission by a factor of at least 100, in particular of at least 1000.

6. The method of any one of claims 2 to 5, wherein the current draw is generated with a peak current corresponding to the maximum current present during the data transmission by RF.

7. The method of any one of claims 1 to 6, wherein steps a) to d) are performed periodically, in particular daily.

8. The method of any one of claims 1 to 7, wherein the predetermined threshold voltage VBₛ represents a threshold voltage which still allows at least one data transmission by RF from said active implantable medical device to another device.

9. The method of any one of the preceding claims, wherein the predetermined threshold voltage VBₛ is higher than a limit voltage VB_{ref} which is representative of an operating limit of the RF communication circuit of said active implantable medical device.

10. The method of any one of claims 2 to 9, wherein the current draw is generated using the RF communication unit of said active implantable medical device.

11. The method of claim 10, wherein generating the current draw comprises generating a carrier wave accompanied or not by activation of at least one part of those functions being required for the data transmission by RF.

12. The method of claim 10 or claim 11, wherein generating the current draw comprises using a connection scan channel to initiate an RF communication of said active implantable medical device with another device, in particular using a primary channel of Bluetooth protocol.

13. The method of any one of claims 1 to 12, comprising a step for determining a residual capacity and/or a residual lifetime estimate of the battery as a function of the measured voltage VBᵢ.

14. The method of claim 13, further comprising a step for transmitting a message as a function of the residual capacity and/or of the residual lifetime estimate.

15. The method of any one of claims 1 to 14,comprising a step for transmitting the value VBᵢ to another device upon request of the other device and/or of the user.

16. The method of any one of claims 1 to 15, comprising a step for authorizing or not an automatic monitoring as a function of VBᵢ.

17. The method of any one of claims 1 to 16, wherein the data transmission by RF from the active medical device is suspended after the predetermined threshold voltage VBₛ has been crossed.

18. An active implantable medical device, in particular a cardiac stimulator, comprising a radiofrequency (RF) unit (24), a battery (26), and a controller (30), wherein the controller (30) is configured for implementing a method (10) of any one of claims 1 to 17.
